(19) Europäisches Patentamt
European Patent Office
Office européen des brevets

(11) Veröffentlichungsnummer: **0 188 810**
**B1**

(12) **EUROPÄISCHE PATENTSCHRIFT**

(45) Veröffentlichungstag der Patentschrift: **31.10.90**

(51) Int. Cl.[5]: **A 61 K 37/02**

(21) Anmeldenummer: **85116530.8**

(22) Anmeldetag: **23.12.85**

(54) **Verwendung von Dipeptidderivaten für die Herstellung von Arzneimitteln zur Verhinderung oder Behandlung posttraumatischer Nervenschäden nach unfallbedingten Rückenmarks- und/oder Gehirntraumen.**

(30) Priorität: **23.01.85 DE 3502041**

(43) Veröffentlichungstag der Anmeldung:
**30.07.86 Patentblatt 86/31**

(45) Bekanntmachung des Hinweises auf die Patenterteilung:
**31.10.90 Patentblatt 90/44**

(84) Benannte Vertragsstaaten:
**AT BE CH DE FR GB IT LI LU NL SE**

(73) Patentinhaber: **Grünenthal GmbH**
**Zieglerstrasse 6**
**D-5100 Aachen (DE)**

(72) Erfinder: **Giertz, Hubert, Prof. Dr. med.**
**Finkenhag 24**
**D-5100 Aachen (DE)**
Erfinder: **Barth, Hans, Priv. Doz. Dr.**
**Taubengasse 12**
**D-5100 Aachen (DE)**
Erfinder: **Flohé, Leopold, Prof. Dr. med.**
**Lenzbachstrasse 24**
**D-5106 Roetgen (DE)**

(56) Entgegenhaltungen:
**FR-A-2 347 338**

**PSYCHONEUROENDOCRIN., Band 10, Nr. 3, Seiten 225-235, 1985, Pergamon Press Ltd, GB; E.C. GRIFFITHS: "Thyrotrophin releasing hormone: endocrine and central effects"**

**N. ENGL. J. MED., Band 305, Nr. 18, 1981, Seiten 1063-1067; A.I. FADEN et al.: "Thyrotropin-releasing hormone improves neurologic recovery after spinal trauma in cats"**

(56) Entgegenhaltungen:
**NEUROLOGY, Band 33, Juni 1983, Seiten 673-678, Cleveland, US; A.I. FADEN et al.: "Comparison of thyrotropin-releasing hormone (TRH), naloxone, and dexamethasone treatments in experimental spinal injury"**

**CIRC. SHOCK, Band 21, Seite 376, 1987, Zusammenfassung Nr. 224; T. McINTOSH et al.: "Beneficial effect of the TRH analog CG-3703 on hypotension and neurological deficit caused by traumatic brain injury in rats"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 188 810 B1**

Courier Press, Leamington Spa, England.

(56) Entgegenhaltungen:

PEPTIDES, Band 4, 1983, Seiten 631-634, Ankho International Inc., US; A.I. FADEN et al.: "Neuropeptides in spinal cord injury: comparative experimental models"

NEUROSCIENCE LETT., Band 42, Nr. 1, 1983, Seiten 67-70, Elsevier Scientific Publishers Ireland Ltd; V.A.D. WEBSTER et al.: "Antinociceptive effects of thyrotrophin-releasing hormone and its analogues in the rat periaqueductal grey region"
Faden, A.I. et al., Neurologie 35 (1985), page 1331-1334

**Beschreibung**

Stumpfe Verletzungen des Rückenmarks und des Gehirns erfolgen relativ oft bei Unfällen, insbesondere Verkehrs- und Sportunfällen. Je nach Ausmaß bzw. Schwere dieser Traumen tritt in der Folge dann eine mehr oder weniger ausgeprägte Symptomatik (posttraumatische Nervenschäden wie insbesondere Querschnittslähmung nach Rückenmarkstraumen bzw. zentrale Lähmungen, Störungen der Gedächtnisfunktion oder andere neurologische Ausfallerscheinungen nach Gehirntraumen) auf, die häufig irreversibel und stets schwer zu therapieren ist. In jüngster Zeit wurde beobachtet, daß bestimmte Verbindungen, wie z. B. das Thyrotropin-Releasing-Hormon (TRH = Pyroglutamyl-histidyl-prolinamid) im Tierexperiment günstige Wirkungen auf die Wiedererlangung der Beweglichkeit nach Rückenmarkstraumen haben, wenn sie innerhalb der ersten 24 Stunden nach dem Trauma verabreicht werden. Weiterhin ist im Tierexperiment eine dosisabhängige günstige Wirkung des TRH auf durch Gehirntraumen bewirkte Änderungen des EEG beobachtet worden. Es wurde auch schon beschrieben, daß beim Menschen aufgrund von funktionellen oder organischen Hirnschäden (wie Cranialtrauma, nach Hirnoperationen, bei Hirntumoren usw.) auftretende Bewußtseinsstörungen durch länger fortgeführte TRH-Verabreichung günstig beeinflußt werden können. Leider wird aber das TRH im Organismus schnell enzymatisch durch Abspaltung des Pyroglutamylrestes sowie durch Desamidierung abgebaut und inaktiviert, so daß es für therapeutische Zwecke praktisch nur in Form kontinuierlicher intravenöser Infusionen in hohen Dosen erfolgreich einsetzbar ist und z. B. eine Verabreichung durch intramuskuläre Injektionen oder peroral lediglich ausnahmsweise in Betracht gezogen werden kann.

Man hat daher bereits u. a. durch chemische Modifikation des TRH-Moleküls versucht, zu Substanzen zu gelangen, die bei weitgehend gleicher biologischer Wirkung die aus der Labilität des TRH resultierenden Nachteile nicht aufweisen. Dabei hat sich jedoch gezeigt, daß die Wirkungsprofile der verschiedenen Abwandlungsprodukte unterschiedlich sind und insbesondere auch nur partiell den TRH-Wirkungen entsprechen. Allerdings wurden diese Befunde nur hinsichtlich der endokrinologischen Effekte und der Wirkungen auf das Zentralnervensystem erhoben. Ob und welche Effekte derartige TRH-Abwandlungsprodukte beispielsweise bei posttraumatischen Nervenschäden und insbesondere auf die Symptomatik nach Rückenmarkstraumen und/oder auf die zentralnervösen Ausfallerscheinungen nach Gehirntraumen haben, ist nicht bekannt.

Es wurde nun gefunden, daß durch Verabreichung von Arzneimitteln, welche Dipeptidderivate der allgemeinen Formel I

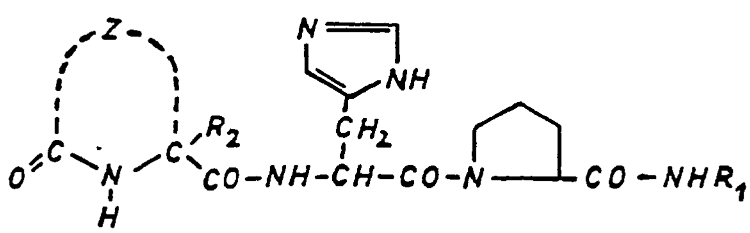

in der $R_1$ ein Wasserstoffatom, einen Alkylrest mit 1 bis 6 Kohlenstoffatomen, einen Cyclohexylrest oder einen Benzylrest bedeutet, und in der Z eine der (mit der durch den Stern markierten Bindung an die CO-Gruppe im Ring angreifenden) Gruppen

(a) 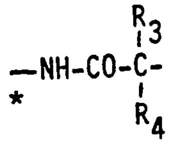 oder (b) 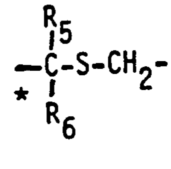

bedeutet, $R_2$ zusammen mit $R_3$ für eine zusätzliche Bindung oder, wenn Z die Gruppe (b) darstellt, für ein Wasserstoffatom steht und in der $R_4$ und $R_5$ für Wasserstoffatome oder für Alkylreste mit 1 bis 3 Kohlenstoffatomen stehen und $R_5$ auch Phenyl bedeuten kann und $R_6$ ein Wasserstoffatom oder Methyl bedeutet

oder deren pharmazeutisch anwendbare Salze mit Säuren enthalten, das akute Auftreten oder die nachfolgende Ausbildung von Nervenschäden, insbesondere bleibender Nervenschäden nach unfallbedingten Rückenmarks- und/oder Gehirntraumen verhindert oder erfolgreich therapiert werden kann.

Für die Herstellung von Arzneimitteln zur Behandlung posttraumatischer Nervenschäden nach unfallbedingten Traumen, insbesondere zur Verhinderung bzw. Behandlung von Querschnittslähmungen nach

Rückenmarkstraumen sowie von zentralnervösen Ausfallerscheinungen nach Gehirntraumen bevorzugte Verbindungen der allgemeinen Formel I entsprechen den allgemeinen Formeln Ia und Ib

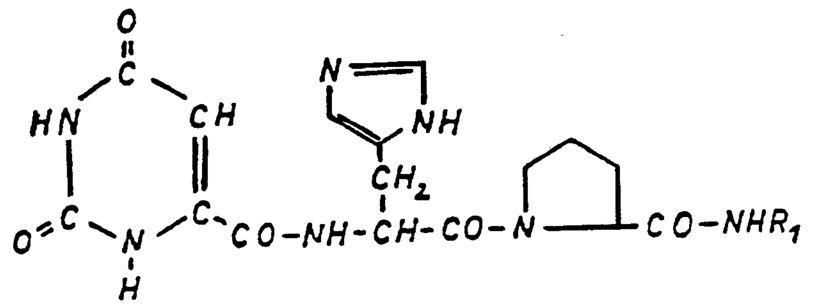

(Ia)

oder

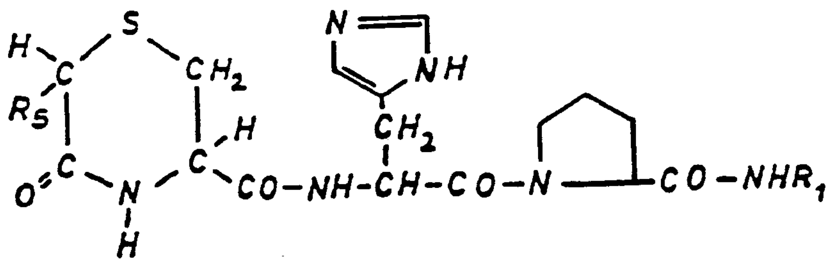

(Ib)

in denen $R_1$ und $R_5$ die oben angegebenen Bedeutungen haben.

Vorzugsweise steht in den allgemeinen Formeln I, Ia bzw. Ib der Rest $R_1$ für ein Wasserstoffatom.

Besonders bevorzugte Verbindungen der allgemeinen Formel I sind Orotyl-L-histidyl-L-prolinamid und 5-Oxo-6-methyl-thiomorpholin-3-(L)-carbonyl-L-histidyl-L-prolinamid.

Die Verbindungen der allgemeinen Formel I und ihre Herstellung sind z. B. von Schwertner et al. in "Structure and Activity of Natural Peptides" (Editors W. Voelter and G. Weitzel) Walter de Gruyter-Verlag, Berlin — New York 1981, Seiten 397—415 sowie z. B. in den deutschen Patentschriften 2 449 167 und 2 615 455 beschrieben worden. Aus diesen Literaturstellen ist bekannt, daß die Verbindungen der allgemeinen Formel I nach parenteraler oder auch oraler Verabreichung lang anhaltende zentral erregende Wirkungen ausüben und nur eine geringe Toxizität besitzen. Aufgrund dieser pharmakologischen Eigenschaften können nach den Angaben der genannten und weiterer Literaturstellen die Verbindungen der allgemeinen Formel I als Psychostimulantien bzw. als Antidepressiva eingesetzt werden.

Die bekannten Indikationsbereiche der Verbindungen der allgemeinen Formel I betreffen also chronische Erkrankungen des Gehirns und nicht etwa akut entstehende Schäden des Zentralnervensystems. Es war daher nicht vorauszusehen, daß die Verbindungen der allgemeinen Formel I zur Behandlung posttraumatischer Nervenschäden nach unfallbedingten Traumen, insbesondere zur Verhinderung bzw. Behandlung von Querschnittslähmungen nach Rückenmarkstraumen und/oder von zentralnervösen Ausfallerscheinungen nach Gehirntraumen erfolgreich einsetzbar sein könnten.

Die Erfindung betrifft daher die Verwendung von Dipeptidderivaten der allgemeinen Formel I für die Herstellung von Arzneimitteln zur Verhinderung oder Behandlung von posttraumatischen Nervenschäden nach unfallbedingten Rückenmarks- und/oder Gehirntraumen.

Die erste Verabreichung der Verbindungen der allgemeinen Formel I enthaltenden Arzneimittel an Patienten mit ZNS-Traumen erfolgt nach Möglichkeit noch am Unfallort, und zwar in Form einer Zubereitung zur Injektion, die eine Menge von z. B. 5—100 mg des Wirkstoffes enthält. Die Weiterbehandlung kann dann im Krankenhaus in Form von Infusionen bzw. Injektionen in die Gefäße, unter die Haut, in die Muskulatur, die Bauchhöhle oder den Liquorraum direkt erfolgen.

Solche zur Infusion bzw. Injektion geeigneten Zubereitungsformen der Verbindungen der allgemeinen Formel I sind an sich bekannt. Sie bestehen beispielsweise aus der in trockener, steriler Form (gegebenenfalls als Lyophilisat) in einem Fläschchen mit durchstechbarem Stopfen abgefüllten Wirkstoffmenge, woraus man durch Zugabe eines geeigneten Lösungsmittels, wie z. B. einer sterilen, isotonischen wässrigen Lösung von Kochsalz, Glucose, Inosit der dergleichen, worin die Verbindungen der allgemeinen Formel I leicht löslich sind, zur Injektion oder Infusion einsetzbare Arzneimittellösungen erhält.

Gut geeignete Applikationsformen sind auch Sprays zur intranasalen oder oralen Applikation bzw. zur Verabreichung der Substanzen über die Bronchien, die in üblicher Form hergestellt werden können.

Da die Verbindungen der allgemeinen Formel I chemisch recht stabil sind, wirft ihre Einarbeitung auch in andere als die bereits genannten pharmazeutischen Zubereitungsformen für den Fachmann keinerlei Probleme auf. Zur Erläuterung sei dennoch aber folgendes gesagt:

Insbesondere zur Aufrechterhaltung der Beispielsweise durch Verabreichung von Infusionsformen

einleitend erreichten Plasmaspiegel kommen auch oral bzw. peroral anwendbare Zubereitungsformen der Verbindungen der allgemeinen Formel I, wie Tabletten, Dragees, Kapseln, Granulate, Tropfen und Säfte bzw. Sirupe in Betracht. Auch diese Zubereitungsformen sind an sich bekannt bzw. können in üblicher Weise hergestellt werden.

So kann man beispielsweise zur Herstellung von Tabletten oder Dragees, die jeweils 20 mg einer Verbindung der allgemeinen Formel I enthalten, so vorgehen, daß 20 g der Wirksubstanz mit 35 g Maisstärke, 10 g kolloidaler Kieselsäure, 5 g Magnesiumstearat und gegebenenfalls Zusätzen von Farbstoffen und/oder Aromen vermischt, granuliert, getrocknet und zu 1000 Tabletten gepreßt werden, die dann gewünschten-falls noch mit einem Überzug versehen bzw. dragiert werden.

Zur Herstellung von jeweils 20 mg der Verbindung der allgemeinen Formel I enthaltenden Kapseln kann man in üblicher Weise z. B. 20 g Wirkstoff mit 376 g Lactose vermischen, das Gemisch nach Befeuchten mit einer wässrigen Lösung von 4 g Gelatine granulieren, trocknen und in 1000 Hartgelatine-Kapseln füllen.

Nasentropfen, die gegebenenfalls auch in Sprayform appliziert werden können, kann man z. B. in an sich bekannter Weise dadurch erhalten, daß man die Verbindung der allgemeinen Formel I in einer isotonen wässrigen Lösung von Natriumchlorid, Mannit, Sorbit oder Inosit etc. löst und z. B. Polyvinylpyrrolidon oder Polyvinylalkohol als Haftmittel und/oder ein Konservierungsmittel, wie z. B. p-Hydroxybenzoesäuremethylester oder Benzylalkohol, zufügt.

Einen Wirkstoff der allgemeinen Formel I enthaltende Suppositorien kann man z. B. durch Schmelzen von 95 g einer handelsüblichen Suppositorienmasse bei 40—45°C, Zugabe von 3 g Salicylsäure oder Mandelsäure, anschließen des Einrühren von 2 g des Wirkstoffes und Einfüllen des Gemisches in Suppositorienformen herstellen.

In vielen Fällen kommen auch percutane Applikationszubereitungen (wie z. B. die Wirkstoffe in einem Depot in gelöster Form, gegebenenfalls unter Zusatz von bekannten, die Hautpenetration fördernden Mitteln wie N-Alkyllactamen oder dergleichen enthaltende Pflaster oder dergleichen) in Betracht.

Die vorstehend beschriebenen peroral, rektal, percutan oder intramuskulär anwendbaren Zubereitungsformen können vorzugsweise in ebenfalls an sich bekannter Weise auch so hergestellt werden, daß daraus nach der Applikation die Verbindung der allgemeinen Formel I verzögert freigesetzt wird, um so über einen längeren Zeitraum (beispielsweise 24 Stunden) eine gleichförmige Versorgung des Patienten mit dem Wirkstoff zu gewährleisten.

Die zur Verhinderung bzw. zur Therapie der Symptomatik nach Rückenmarks- und/oder Gehirntraumen erforderlichen Dosen an Verbindungen der allgemeinen Formel I richten sich — abgesehen von der Applikationsart — insbesondere nach dem Grad der Schädigung und sind von Arzt individuell zu ermitteln bzw. festzusetzen. Als Anhaltspunkt kann davon ausgegangen werden, daß der in Betracht zu ziehende Dosierungsbereich für die Applikation mit Hilfe der infusions- bzw. Injektionstechnik bei etwa 1 bis 300 mg pro Tag liegt.

Die überraschende Wirkung der Verbindungen der allgemeinen Formel I bzw. von diese enthaltenden pharmazeutischen Zubereitungsformen könnte tierexperimentell z. B. wie folgt gezeigt werden:

Narkotisierten Katzen wurde ein Rückenmarkstrauma gesetzt, indem man aus 30 cm Höhe ein 20 g-Gewicht auf eine 10 $mm^2$ große Aufprallplatte, die am freigelegten Rückenmark anlag, fallen ließ. Jeweils nach einer Stunde bzw. nach 3 Stunden erhielten die Tiere eine (schnelle) intravenöse Injektion von 0,2 mg/kg Orotyl-L-histidyl-L-prolinamid, gelöst in 0,5 ml physiologischer Kochsalzlösung bzw. als Vergleichssubstanz, ebenfalls in 0,5 ml Kochsalzlösung gelöst, 1,0 mg/kg L-Pyro-2-amino-adipyl-L-histidyl-thiazolin-4-carbonsäureamid (diese nachstehend als "Substanz A" bezeichnete Verbindung wurde in der Literatur häufig als TRH-Abwandlungsprodukt beschrieben und vergleichend zu dem genannten Orotyl-L-histidyl-L-prolinamid untersucht, siehe z. B. Metcalf in "Thyrotropin-Releasing Hormone" (Editors E. C. Griffiths and G. W. Bennett) Raven Press, New York 1983, 315—326. Eine Kontrollgruppe erhielt Injektionen von jeweils 0,5 ml physiologischer Kochsalzlösung.

Die neurologischen Funktionen wurden für einen Zeitraum von 6 Wochen wöchentlich überprüft und nach einem vorgegebenen Schema klassifiziert. Dabei zeigte sich, daß nach 6 Wochen die neurologische Funktion bzw. die Extremitätenmotorik bei den mit Orotyl-L-histidyl-L-prolinamid behandelten Tieren signifikant höher ausgeprägt war als nach Verabreichung der Substanz A bzw. der Kochsalzlösung und daß die Substanz A keinen signifikanten Effekt auf die Wiedererlangung der motorischen Funktion hatte. Dieser Befund beweist zugleich, daß aus der Wirkung von TRH-Abwandlungsprodukten auf das Zentralnervensystem (die ja der Substanz A in ausgeprägtem Maße zukommt) kein Rückschluß auf ihre Wirksamkeit für die Verhinderung oder Therapie von posttraumatischen Nervenschäden (hier insbesondere gezeigt am Modell der Querschnittssymptomatik nach Rückenmarkstraumen) möglich ist, d. h. die ermittelte ausgeprägte Wirkung des Orotyl-L-histidyl-L-prolinamids, also einer Verbindung der allgemeinen Formel I, aufgrund der dafür bekannten pharmakologischen Eigenschaften nicht zu erwarten oder vorherzusehen war.

Ähnliche Resultate wie die oben für die Therapie mit Orotyl-L-histidyl-L-prolinamid beschriebenen wurden auch durch Verabreichung von Zubereitungsformenn anderer Verbindungen der allgemeinen Formel I erzielt. Die dabei erfolgreich eingesetzten Dosierungen betrugen z. B. 0,1 mg/kg, 0,2 mg/kg, 0,5 mg/kg und 1 mg/kg. Aufgrund der bekannten geringen Toxizität der Verbindungen der allgemeinen Formel I können diese abei ohne weiteres auch in höheren Dosierungen angewendet und/oder gewünschtenfalls

öfter als im beschriebenen Experiment (z. B. 3- bis 5-mal täglich für einen längeren Zeitraum) verabreicht werden.

Dem oben für das Tierexperiment beschriebenen Effekt vergleichbare Wirkungen wurden auch nach Verabreichung von Substanzen der allgemeinen Formel I enthaltenden Arzneimitteln an menschliche Unfallopfer, die eine stumpfe Verletzung des Rückenmarks erlitten hatten und bei denen aufgrund des Ausmaßes der erlittenen Schädigungen das Auftreten einer Querschnittslähmung zu befürchten gewesen war, beobachtet. Hierbei konnten aber selbstverständlich aus ethischen Gründen Kontrollversuche z. B. mit der Substanz A oder durch Gabe lediglich von physiologischer Kochsalzlösung nicht durchgeführt werden.

**Patentansprüche**

1. Verwendung von Dipeptidderivaten der allgemeinen Formel I

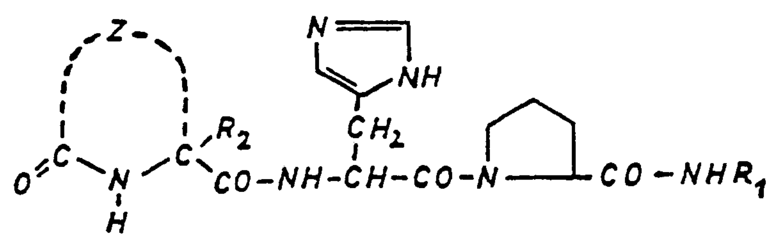

in der $R_1$ ein Wasserstoffatom, einen Alkylrest mit 1 bis 6 Kohlenstoffatomen, einen Cyclohexylrest oder einen Benzylrest bedeutet, und in der Z eine der (mit der durch einen Stern markierten Bindung an die CO-Gruppe im Ring angreifenden) Gruppen

(a) 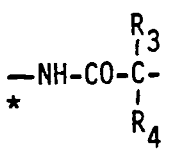 oder (b) 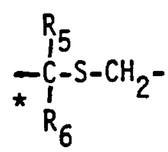

bedeutet, $R_2$ zusammen mit $R_3$ für eine zusätzliche Bindung oder, wenn Z die Gruppe (b) darstellt, für ein Wasserstoffatom steht und in der $R_4$ und $R_5$ für Wasserstoffatome oder für Alkylreste mit 1 bis 3 Kohlenstoffatomen stehen und $R_5$ auch Phenyl bedeuten kann und $R_6$ ein Wasserstoffatom oder Methyl ist

oder deren pharmazeutisch anwendbaren Salzen mit Säuren,

für die Herstellung von Arzneimitteln zur Verhinderung oder Behandlung posttraumatischer Nervenschäden nach unfallbedingten Rükenmarks- und/oder Gehirntraumen.

2. Verwendung von Dipeptidderivaten der allgemeinen Formel I aus Anspruch 1 oder deren pharmazeutisch anwendbaren Salzen mit Säuren für die Herstellung von Arzneimitteln zur Verhinderung oder Behandlung von Querschnittslähmungen nach Rückenmarkstraumen und/oder von zentralnervösen Ausfallerscheinungen nach Gehirntraumen gemäß Anspruch 1.

3. Verwendung von Dipeptidderivaten der allgemeinen Formel I aus Anspruch 1 oder deren pharmazeutisch anwendbaren Salzen mit Säuren für die Herstellung von Arzneimitteln zur Verhinderung oder Behandlung von Querschnittslähmungen nach unfallbedingten Rückenmarkstraumen gemäß Ansprüchen 1 und 2.

4. Verwendung von Dipeptidderivaten der allgemeinen Formel

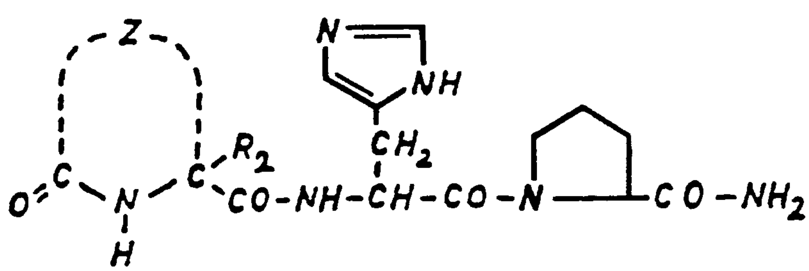

in der $R_2$ und Z die gleiche Bedeutung wie in Anspruch 1 haben

oder deren pharmazeutisch anwendbaren Salzen mit Säuren

für die Herstellung von Arzneimitteln zur Verhinderung oder Behandlung posttraumatischer Nervenschäden nach unfallbedingten Traumen, insbesondere zur Verhinderung oder Behandlung von Querschnittslähmungen nach Rückenmarkstraumen und/oder von zentralnervösen Ausfallerscheinungen nach Gehirntraumen gemäß Ansprüchen 1 bis 3.

5. Verwendung von Dipeptidderivaten der allgemeinen Formel Ia

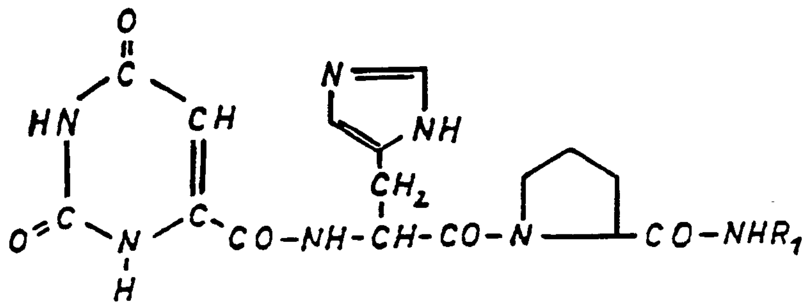

(Ia)

worin $R_1$ die gleiche Bedeutung wie in Anspruch 1 hat

oder deren pharmazeutisch anwendbaren Salzen mit Säuren

für die Herstellung von Arzneimitteln zur Verhinderung oder Behandlung posttraumatischer Nervenschäden nach unfallbedingten Traumen, insbesondere zur Verhinderung oder Behandlung von Querschnittslähmungen nach Rückenmarkstraumen und/oder von zentralnervösen Ausfallerscheinungen nach Gehirntraumen gemäß Ansprüchen 1 bis 4.

6. Verwendung von Dipeptidderivaten der allgemeinen Formel Ib

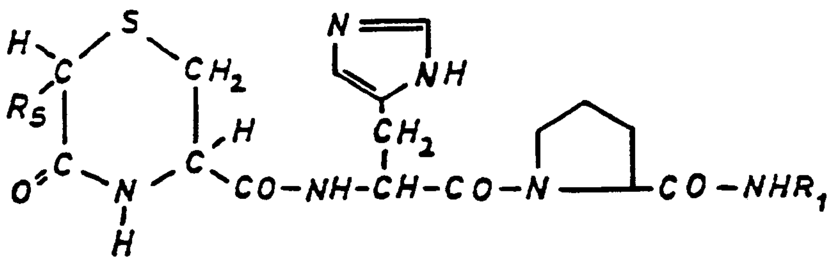

(Ib)

worin $R_1$ und $R_5$ die gleiche Bedeutung wie in Anspruch 1 haben

oder deren pharmazeutisch anwendbaren Salzen mit Säuren

für die Herstellung von Arzneimitteln zur Verhinderung oder Behandlung posttraumatischer Nervenschäden nach unfallbedingten Traumen, insbesondere zur Verhinderung oder Behandlung von Querschnittslähmungen nach Rückenmarkstraumen und/oder von zentralnervösen Ausfallerscheinungen nach Gehirntraumen gemäß Ansprüchen 1 bis 4.

7. Verwendung von Orotyl-L-histidyl-L-prolinamid oder eines von dessen pharmazeutisch anwendbaren Salzen mit Säuren für die Herstellung von Arzneimitteln zur Verhinderung oder Behandlung posttraumatischer Nervenschäden nach unfallbedingten Traumen, insbesondere zur Verhinderung oder Behandlung von Querschnittslähmungen nach Rückenmarkstraumen und/oder von zentralnervösen Ausfallerscheinungen nach Gehirntraumen gemäß Ansprüchen 1 bis 5.

8. Verwendung von 5-Oxo-6-methyl-thiomorpholin-3-(L)-carbonyl-L-histidyl-L-prolinamid oder eines von dessen pharmazeutisch anwendbaren Salzen mit Säuren für die Herstellung von Arzneimitteln zur Verhinderung oder Behandlung posttraumatischer Nervenschäden nach unfallbedingten Traumen, insbesondere zur Verhinderung oder Behandlung von Querschnittslähmungen nach Rückenmarkstraumen und/oder von zentralnervösen Ausfallerscheinungen nach Gehirntraumen gemäß Ansprüchen 1 bis 4 und 6.

9. Verwendung von Dipeptidderivaten der allgemeinen Formeln aus Ansprüchen 1 und 4 bis 6 oder deren pharmazeutisch anwendbaren Salzen für die Herstellung von durch Infusion oder Injektion zu verabreichenden Arzneimitteln gemäß Ansprüchen 1—8 zur Verhinderung oder Behandlung posttraumatischer Nervenschäden nach unfallbedingten Traumen, insbesondere nach unfallbedingten Rückenmarks- und/oder Gehirntraumen.

10. Verwendung von Dipeptidderivaten der allgemeinen Formeln aus Ansprüchen 1 und 4 bis 6 oder deren pharmazeutisch anwendbaren Salzen mit Säuren für die Herstellung von Arzneimitteln zur percutanen Wirkstoffzuführung für die Verhinderung oder Behandlung posttraumatischer Nervenschäden nach unfallbedingten Traumen, insbesondere nach unfallbedingten Rückenmarks- und/oder Gehirntraumen, gemäß Ansprüchen 1—8.

11. Verwendung von Dipeptidderivaten der allgemeinen Formeln aus Ansprüchen 1 und 4 bis 6 oder deren pharmazeutisch anwendbaren Salzen für die Herstellung oral, peroral oder rektal anzuwendender Arzneimittel gemäß Ansprüchen 1—8 zur Verhinderung oder Behandlung posttraumatischer Nervenschäden nach unfallbedingten Traumen, insbesondere zur Verhinderung bzw. Behandlung von Querschnittslähmungen nach Rückenmarkstraumen und/oder von zentralnervösen Ausfallerscheinungen nach Gehirntraumen.

**Revendications**

1. Utilisation de dérivés dipeptidiques de formule générale I

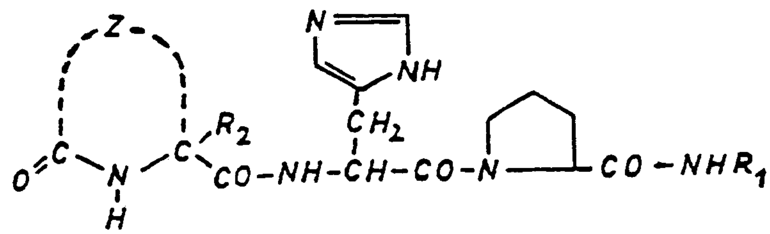

(I)

dans laquelle $R_1$ désigne un atome d'hydrogène, un reste alkyle ayant 1 à 6 atomes de carbone, un reste cyclohexyle ou un reste benzyle, et dans laquelle Z est l'un des groupes

(a) 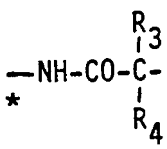 oder (b) 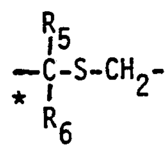

(s'attachant par la liaison marquée d'un astérisque au groupe CO du noyau), $R_2$ forme conjointement avec $R_3$ une liaison supplémentaire ou bien, lorsque Z représente le groupe (b), désigne un atome d'hydrogène, et dans laquelle $R_4$ et $R_5$ désignent des atomes d'hydrogène ou des restes alkyle ayant 1 à 3 atomes de carbone et $R_5$ peut aussi représenter un groupe phényle, et $R_6$ est un atome d'hydrogène ou un groupe méthyle

ou de leurs sels d'acides acceptables du point de vue pharmaceutique

pour la préparation de médicaments destinés à prévenir ou à traiter des lésions nerveuses post-traumatiques après des traumatismes de la moelle épinière et/ou du cerveau causés par un accident.

2. Utilisation de dérivés dipeptidiques de formule générale I suivant la revendication 1 ou de leurs sels d'acides acceptables du point de vue pharmaceutique pour la préparation de médicaments destinés à prévenir ou à traiter des paralysies transverses après des traumatismes de la moelle épinière et/ou des défaillances du système nerveux central après des traumatismes du cerveau, suivant la revendication 1.

3. Utilisation de dérivés dipeptidiques de formule générale I suivant la revendication 1 ou de leurs sels d'acides acceptables du point de vue pharmaceutique pour la préparation de médicaments destinés à prévenir ou à traiter des paralysies transverses après des traumatismes de la moelle épinière causés par un accident, suivant les revendications 1 et 2.

4. Utilisation de dérivés dipeptidiques de formule générale

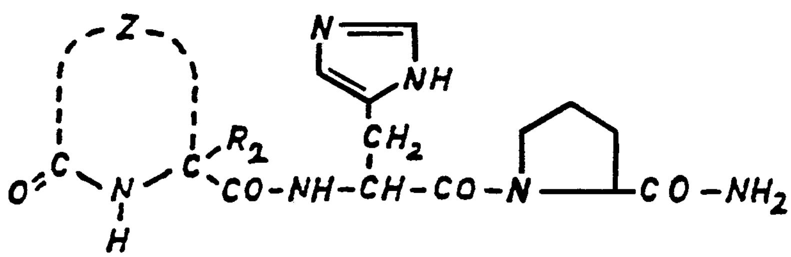

dans laquelle $R_2$ et Z ont la même définition que dans la revendication 1,

ou de leurs sels d'acides acceptables du point de vue pharmaceutique

pour la préparation de médicaments destinés à prévenir ou à traiter des lésions nerveuses post-traumatiques à la suite de traumatismes occasionnés par un accident, notamment pour prévenir ou traiter des paralysies transverses après des traumatismes de la moelle épinière et/ou des défaillances du système

nerveux central après des traumatismes du système nerveux central après des traumatismes du cerveau suivant les revendications 1 à 3.

5. Utilisation de dérivés dipeptidiques de formule générale la

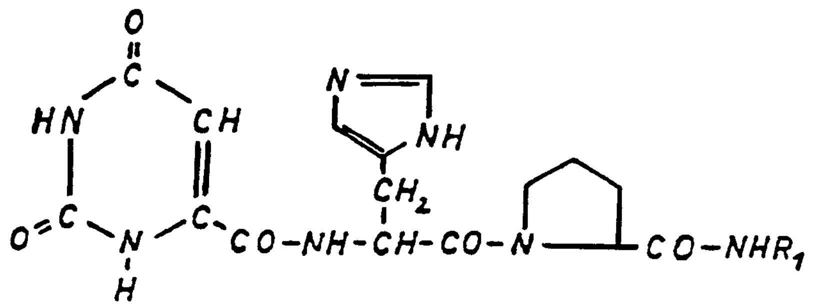

(Ia)

dans laquelle $R_1$ a la même définition que dans la revendication 1

ou de leurs sels d'acides acceptables du point de vue pharmaceutique

pour la préparation de médicaments destinés à prévenir ou à traiter des lésions nerveuses post-traumatiques après des traumatismes occasionnés par un accident, notamment pour prévenir ou traiter des paralysies transverses après des traumatismes de la moelle épinière et/ou des défaillances du système nerveux central après des traumatismes du cerveau suivant les revendications 1 à 4.

6. Utilisation de dérivés dipeptidiques de formule générale Ib

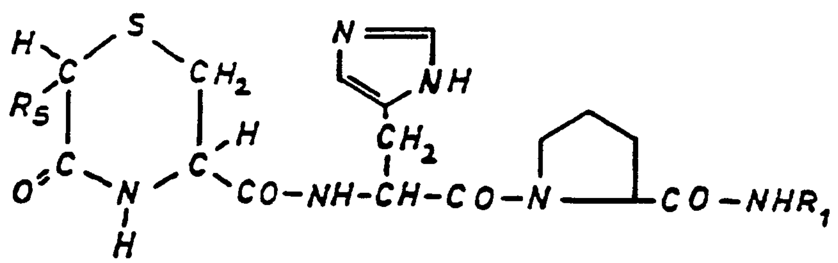

(Ib)

dans laquelle $R_1$ et $R_5$ ont la mème définition que dans la revendication 1

ou de leurs sels d'acides acceptables du point de vue pharmaceutique

pour la préparation de médicaments destinés à prévenir ou à traiter des lésions nerveuses post-traumatiques à la suite de traumatismes causés par un accident, notamment pour prévenir ou traiter des paralysies transverses après des traumatismes de la moelle épinière et/ou des défaillances du système nerveux central après des traumatismes du cerveau, suivant les revendications 1 à 4.

7. Utilisation de l'orotyl-L-histidyl-L-prolinamide ou de l'un de ses sels d'acides acceptables du point de vue pharmaceutique pour la préparation de médicaments destinés à prévenir ou à traiter des lésions nerveuses post-traumatiques à la suite de traumatismes causés par un accident, notamment pour prévenir ou traiter des paralysies transverses après des traumatismes de la moelle épinière et/ou des défaillances du système nerveux central après des traumatismes du cerveau, suivant les revendications 1 à 5.

8. Utilisation du 5-oxo-6-méthyl-thiomorpholino-3-(L)-carbonyl-L-histidyl-L-prolinamide ou de l'un de ses sels d'acides acceptables du point de vue pharmaceutique pour la préparation de médicaments destinés à prévenir ou à traiter des lésions nerveuses post-traumatiques à la suite de traumatismes causés par un accident, notamment pour prévenir ou traiter des paralysies transverses après des traumatismes de la moelle épinière et/ou des défaillances du système nerveux central après des traumatismes du cerveau, suivant les revendications 1 à 4 et 6.

9. Utilisation de dérivés dipeptidiques de formules générales suivant les revendications 1 et 4 à 6 ou de leurs sels pharmaceutiquement acceptables pour la préparation de médicaments destinés à être administrés par infusion ou par injection suivant les revendications 1 à 8 pour prévenir ou traiter des lésions nerveuses post-traumatiques à la suite de traumatismes causés par un accident, notamment à la suite de traumatismes de la moelle épinière et/ou du cerveau causés par un accident.

10. Utilisation de dérivés dipeptidiques de formules générales suivant les revendications 1 et 4 à 6 ou de leurs sels d'acides acceptables du point de vue pharmaceutique pour la préparation de médicaments destinés à un apport percutané de substance active pour prévenir ou traiter des lésions nerveuses post-traumatiques à la suite de traumatismes causés par un accident, notamment à la suite de traumatismes de la moelle épinière et/ou du cerveau causés par un accident, suivant les revendications 1 à 8.

11. Utilisation de dérivés dipeptidiques de formules générales suivant les revendications 1 et 4 à 6 ou de leurs sels acceptables du point de vue pharmaceutique pour la préparation de médicaments à administrer par voie orale, perorale ou rectale suivant les revendications 1 à 8, destinés à prévenir ou à

traiter des lésions nerveuses post-traumatiques à la suite de traumatismes causés par un accident, notamment pour prévenir et traiter des paralysies transverses après des traumatismes de la moelle épinière et/ou des défaillances du système nerveux central à la suite de traumatismes du cerveau.

**Claims**

1. Use of dipeptide derivatives of the general formula I

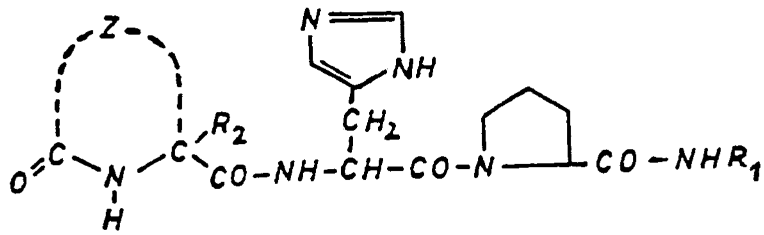

(I)

in which $R_1$ stands for a hydrogen atom, an alkyl residue with 1 to 6 carbon atoms, a cyclohexyl residue or a benzyl residue, and which Z stands for one of the groups

(a) 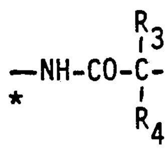 oder (b) 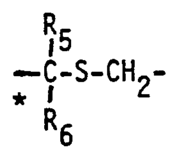

(acting with the bond marked by a star on the CO-group in the ring), $R_2$ together with $R_3$ stands for an additional bond or, if Z represents group (b), for a hydrogen atom and in which $R_4$ and $R_5$ stand for hydrogen atoms or for alkyl residues with 1 to 3 carbon atoms and $R_5$ can also mean phenyl and $R_6$ is a hydrogen atom or methyl

or their pharmaceutically available salts with acids

for the production of medicines for prevention or treatment of post-traumatic nerve damage after spinal cord and/or brain trauma caused by an accident.

2. Use of dipeptide derivatives of the general formula I in Claim 1 or their pharmaceutically available salts with acids for the production of medicines for prevention or treatment of paraplegia after spinal cord trauma and/or central nervous deficiency symptoms after brain trauma according to Claim 1.

3. Use of dipeptide derivatives of the general formula I in Claim 1, or their pharmaceutically available salts with acids for the production of medicines for prevention or treatment of paraplegia after spinal cord traumas, according to Claims 1 and 2.

4. Use of dipeptide derivatives of the general formula I

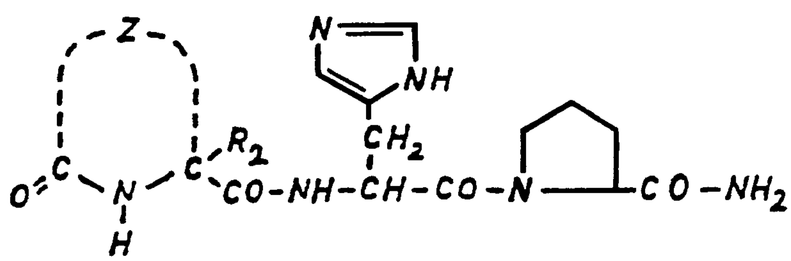

in which $R_2$ and Z have the same meaning as in Claim 1,

or their pharmaceutically available salts with acids,

for the production of medicines to prevent or treat post traumatic nerve damage after accident-related

traumas, especially for the prevention or treatment of paraplegia after spinal cord traumas and/or central nervous deficiency symptoms after brain traumas, according to Claims 1 to 3.

5. Use of dipeptide derivatives of the general formula Ia

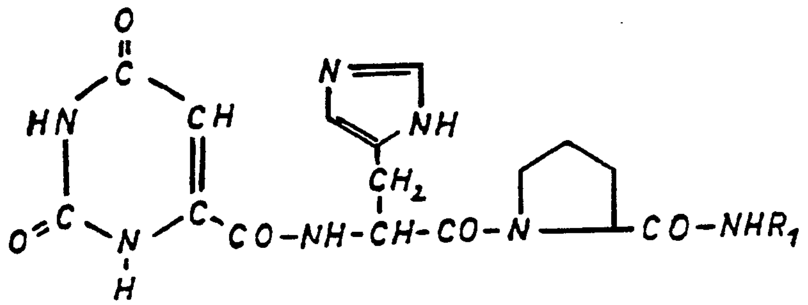

(Ia)

in which $R_1$ has the same meaning as in Claim 1,

or their pharmaceutically available salts with acids,

for the production of medicines for the prevention or treatment of post traumatic nerve damage after accident-related traumas, especially for the prevention or treatment of paraplegia after spinal cord traumas and/or of central nervous deficiency symptoms after brain traumas according to Claims 1 to 4.

6. Use of dipeptide derivatives of the general formula Ib

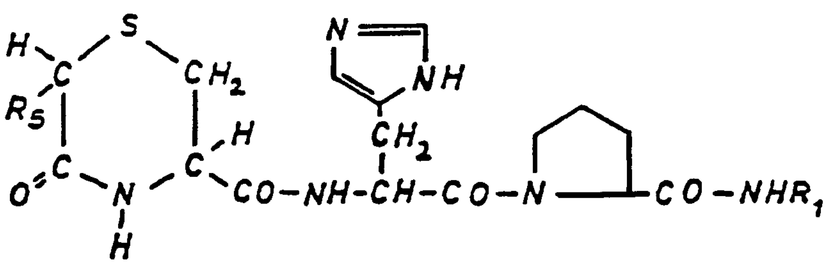

(Ib)

in which $R_1$ and $R_5$ have the same meaning as in Claim 1,

or their pharmaceutically available salts with acids,

for the production of medicines for the prevention or treatment of post traumatic nerve damage after accident-related traumas, especially for the prevention or treatment of paraplegia after spinal cord traumas and/or of central nervous deficiency symptoms after brain traumas according to Claims 1 to 4.

7. Use of orotyl-L-histidyl-L-prolinamide or one of its pharmaceutically available salts with acids for the manufacture of medicines for the prevention or treatment of post traumatic nerve damage after accident-related traumas, in particular for the prevention or treatment of paraplegia after spinal cord traumas and/or of central nervous deficiency symptoms after brain traumas according to Claims 1 to 5.

8. Use of 5-oxo-6-methyl-thiomorpholin-3-(L)-carbonyl-L-histidyl-L-prolinamide or one of its pharmaceutically available salts with acids for the production of medicines for the prevention or treatment of post traumatic nervous damage after accident-related traumas, especially for the prevention or treatment of paraplegias after spinal cord traumas and/or of central nervous deficiency symptoms after brain traumas according to Claims 1 to 4 and 6.

9. Use of dipeptide derivatives of the general formula in Claims 1 and 4 to 6 or their pharmaceutically available salts for the production of medicines to be administered by infusion or injection according to Claims 1—8, for the prevention or treatment of post traumatic nerve damage after accident-related traumas, especially after accident-related spinal cord and/or brain traumas.

10. Use of dipeptide derivatives of the general formula in claims 1 and 4 to 6 or their pharmaceutically available salts with acids for the production of medicines for percutaneous supply of active material for the prevention or treatment of post traumatic nerve damage after accident-related traumas, especially after accident-related spinal cord and/or brain traumas, according to claims 1—8.

11. Use of dipeptide derivatives of the general formula of Claims 1 and 4 to 6 or their pharmaceutically available salts for the production of orally, perorally or rectally applied medicines according to Claims 1—8 for the prevention or treatment of post traumatic nerve damage after accident-related traumas, especially for the prevention or treatment of paraplegia after spinal cord traumas and/or of central nervous deficiency symptoms after brain traumas.